# EUROPÄISCHE PATENTANMELDUNG

(11) **EP 1 174 155 A2**
(43) Veröffentlichungstag der Anmeldung: **23.01.2002**
(21) Anmeldenummer: 01114475.5
(22) Anmeldetag: 15.06.2001
(51) Int. Cl.: A61L 11/00

(54) **Vorrichtung zum Zerkleinern und Sterilisieren von insbesondere medizinischem Abfall**

(30) Priorität: 20.07.2000 DE 20012608 U
(71) Anmelder: C.M.S. S.P.A., Uffici Commerciali, 41054 Marano S/P (MO) (IT)
(72) Erfinder: Salda, Luciano, 41058 Vignola (MO) (IT)
(74) Vertreter: Grünecker, Kinkeldey, Stockmair & Schwanhäusser Anwaltssozietät

(57) **Zusammenfassung**

Die Vorrichtung dient zum Verkleinern und Sterilisieren von insbesondere medizinischem Abfall. Zwischen einer Eingabe- und einer Ausgabeöffnung verläuft ein Verarbeitungspfad, der einen Eingaberaum, eine Zerkleinerungseinrichtung, eine Sterilisiereinrichtung und eine Verpackungseinrichtung aufweist. Eine Vielzahl von UV-Lampen ist entlang des Pfades angeordnet, wenigstens eine davon beleuchtet die Zerkleinerungseinrichtung.

## Beschreibung

Die Erfindung betrifft eine Vorrichtung zum Zerkleinern und Sterilisieren von insbesondere medizinischem Abfall mit einer Eingabe- und einer Ausgabeöffnung und einem zwischen diesen zu durchlaufenden Verarbeitungspfad, entlang welchem nacheinander wenigstens ein Eingaberaum, eine Zerkleinerungseinrichtung, eine Sterilisiereinrichtung und eine Verpackungseinrichtung angeordnet sind.

Eine solche Vorrichtung ist aus der Praxis bekannt. Der zugeführte Abfall besteht aus Krankenhausabfällen, die sich sowohl aus organischen als auch anorganische Bestandteile enthalten. Um zu verhindern, dass beispielsweise gebrauchte Spritzen, bakteriell oder dergleichen verseuchte Abfälle aus dem Krankenhaus ohne weitere Aufbereitung entsorgt werden, erfolgt insbesondere eine Zerkleinerung dieser Abfälle und eine anschließende Sterilisierung. Durch die Zerkleinerung soll verhindert werden, dass gebrauchte Spritzen nochmals benutzt werden und gleichzeitig soll der Abfall zur anschließenden Sterilisierung vorbereitet werden. Dies gilt ebenfalls für entsprechende organische Abfälle.

Nach der Sterilisierung wird der Abfall verpackt und dann zusammen mit anderem Hausmüll oder dergleichen in an sich bekannter Weise entsorgt.

Die vorbekannte Vorrichtung arbeitet durchaus zufriedenstellend bei der Volumenreduzierung solcher Abfälle und der Sterilisierung eines solchen Abfalls.

Um allerdings solche Abfälle weniger chemisch zu belasten und zu verhindern, dass gegebenenfalls bei der Sterilisierung Bestandteile der Abfälle nicht ausreichend behandelt werden, liegt der Erfindung die Aufgabe zugrunde, in konstruktiv einfacher Weise die Sterilisierung zu verbessern und gleichzeitig im Wesentlichen während der gesamten Aufbereitung der Abfälle durchzuführen.

Diese Aufgabe wird im Zusammenhang mit dem Merkmal des Oberbegriffs des Anspruchs 1 dadurch gelöst, dass eine Anzahl von UV-Lampen (Ultraviolett-Lampen) entlang des Verarbeitungspfades angeordnet sind, von denen wenigstens eine UV-Lampe die Zerkleinerungseinrichtung beleuchtet.

Durch die UV-Lampen kann bereits eine Sterilisierung im Bereich des Eingaberaums, noch vor der Zerkleinerungseinrichtung, während der Zerkleinerung und auch noch bei der Weiterführung der zerkleinerten Abfälle zur Sterilisiereinrichtung erfolgen. Es ist ebenfalls möglich, noch in der Sterilisiereinrichtung UV-Lampen zu verwenden.

Insbesondere im Zusammenhang mit der Zerkleinerungseinrichtung, die verschiedene Abfälle aufschließt und der UV-Bestrahlung zugänglich macht, ist die Anordnung einer solchen UV-Lampe von Vorteil. Dabei wird die sterilisierende Wirkung des UV-Lichtes nicht nur durch die Zerkleinerung der Abfälle intensiviert, sondern auch durch die Mischung und Drehung der Abfälle beim Zerkleinerungsvorgang.

Um zu verhindern, dass UV-Licht aus der Vorrichtung austritt oder dass ein Benutzer der Vorrichtung sich beim Einfüllen des Abfalls an Einrichtungen der Vorrichtung verletzt, kann die Eingabeöffnung durch eine Zugangstür verschließbar sein. Diese ist durch den Benutzer vor Einlegen des Abfalls gegebenenfalls zu öffnen und anschließend wieder zu schließen. Es kann eine entsprechende Sicherheitsschaltung vorgesehen sein, die die UV-Lampen und die weiteren Einrichtungen der Vorrichtung erst dann einschaltet, wenn die Zugangstür wieder verschlossen ist.

Um die Vorrichtung bedienfreundlicher zu gestalten, kann die Zugangstür automatisch öffnen und schließen. Dabei kann die Betätigung der Zugangstür beispielsweise durch Knopfdruck erfolgen.

Um bei einem Aufstellen der erfindungsgemäßen Vorrichtung in einem öffentlichen Bereichen einen unberechtigten Zugang zu verhindern, öffnet sich die Zugangstür nur bei Auslösen einer entsprechenden Zugangsberechtigungseinrichtung. Diese bildet ein Sicherheitssystem, das den Zugang von unberechtigten Benutzern verhindern soll. Die Zugangsberechtigungseinrichtung kann beispielsweise durch eine Tastatur gebildet sein, über die ein entsprechender Zutrittscode eingegeben werden muss. Weitere Beispiele für eine solche Zugangsberechtigungseinrichtung sind ein Magnetkartenlesegerät, eine Schließeinrichtung, die beispielsweise nur mit dem entsprechenden Schlüssel eine Freischaltung der Vorrichtung ermöglicht, oder dergleichen.

Um die Zugangstür vollkommen bedienungsfrei auszubilden, kann der Zugangstür ein Anwesenheitssensor zugeordnet sein. Wird durch diesen die Anwesenheit eines Benutzers in einem bestimmten Bereich vor der Zugangstür der Vorrichtung festgestellt, öffnet sich die Zugangstür automatisch. Nachdem der Benutzer den entsprechenden Bereich vor der Zugangstür wieder verlassen hat, was ebenfalls durch den Anwesenheitssensor feststellbar ist, schließt die Zugangstür automatisch.

Um die weitere Verarbeitung des Abfalls räumlich vom Eingaberaum zu trennen, kann diesem ein Verdichtungsraum nachgeordnet sein.

Um beim Einstellen des Abfalls durch den Benutzer zu verhindern, dass dieser in den Verdichtungsraum und damit nahe an die Einrichtungen der Vorrichtung kommt, kann zwischen Eingaberaum und Verdichtungsraum eine automatisch betätigbare Zwischentür angeordnet sein. Diese öffnet sich beispielsweise erst dann, wenn die Zugangstür nach Einstellen des Abfalls wieder geschlossen ist.

Eine einfache und raumsparende Ausbildung der Türen kann darin gesehen werden, wenn Zugangstür und/oder Zwischentür als Schiebetüren ausgebildet sind.

Es besteht die Möglichkeit, dass Eingaberaum und Verdichtungsraum untereinander oder schräg übereinander angeordnet sind, wobei bei Öffnen der Zwischentür der in den Eingaberaum eingestellte Abfall selbsttätig in Richtung Verdichtungsraum rutscht. Ebenso besteht die Möglichkeit, dass Eingaberaum und Verdichtungsraum nebeneinander angeordnet sind, d.h. im Wesentlichen in gleicher Höhe. Dies hat den Vorteil, dass die Bauhöhe der erfüllungsgemäßen Vorrichtung geringer ist.

Um in einfacher Weise den Abfall zwischen Eingaberaum und Verdichtungsraum zu transportieren, kann eine Transfereinrichtung zum Transport der Abfälle zwischen beiden Räumen vorgesehen sein. Eine solche Transfereinrichtung kann durch einen Bandförderer, einen verschiebbaren Stempel oder dergleichen gebildet sein. Eine schnelle Art der Beförderung des Abfalls kann darin gesehen werden, wenn die Transfereinrichtung eine zwischen einer Aufnahmestellung und einer Kippstellung verschwenkbare Kippebene aufweist. Diese ist zum Einstellen des Abfalls bei geöffneter Zugangstür in ihrer Aufnahmestellung angeordnet. Dann kann der Benutzer den Abfall auf die Kippebene aufstellen. Nach Schließen der Zugangstür und Öffnen der Zwischentür wird dann die Kippebene durch eine entsprechende Antriebseinrichtung in ihre Kippstellung verschwenkt. In dieser rutscht der Abfall von der Kippebene in Richtung Zerkleinerungseinrichtung.

Um gegebenenfalls den Abfall zur weiteren Verarbeitung bereits vorzuverdichten oder um die Zerkleinerungseinrichtung nur nach mehrfacher Eingabe von Abfall in die erfindungsgemäße Vorrichtung zu betätigen, kann eine Presseinrichtung im Verdichtungsraum angeordnet sein. Der in den Verdichtungsraum gelangte Abfall wird durch diese vorverdichtet und erst wenn der Verdichtungsraum ausreichend gefüllt ist, wird der Abfall der Zerkleinerungseinrichtung zugeführt. Dadurch wird vermieden, dass die Zerkleinerungseinrichtung zu häufig in Betrieb ist. Außerdem kann durch die Presseinrichtung der in der Regel locker in den Eingaberaum eingefüllte Abfall vorverdichtet werden, wodurch die Zerkleinerungseinrichtung effektiver arbeiten kann.

Bei einem einfachen Ausführungsbeispiel kann die Presseinrichtung wenigstens ein verschwenkbares, insbesondere plattenförmiges Presselement aufweisen, welches den Abfall in Richtung Zerkleinerungseinrichtung drückt.

Um die Vorrichtung während des Betriebs der Zerkleinerungseinrichtung für weiteren Abfall zugänglich zu machen und solchen vom Eingaberaum zum Verdichtungsraum überführen zu können und gleichzeitig den durch die Presseinrichtung vorverdichteten Abfall weiter zu verdichten und in Richtung Zerkleinerungseinrichtung zu drücken, kann eine Verdichtereinrichtung zur Komprimierung von in Richtung Zerkleinerungseinrichtung durch die Presseinrichtung gedrückten Abfall der Zerkleinerungseinrichtung vorgeordnet sein.

Im einfachsten Fall kann die Verdichtereinrichtung eine in Richtung Zerkleinerungseinrichtung bewegbare Verdichtungsplatte aufweisen, die sich in Richtung Zerkleinerungseinrichtung hin- und herbewegt.

Um die Vorrichtung kompakt zu gestalten, können Zerkleinerungseinrichtung und Verdichtungseinrichtung vertikal übereinander angeordnet sein und die Verdichtungsplatte kann vertikal bewegbar sein.

Die Zerkleinerungseinrichtung kann aus einer Reihe von Walzen gebildet sein, die beispielsweise entlang der Walzenachse angeordnete Messerscheiben aufweisen. Weiterhin können solche Walzen oder die entsprechenden Messerscheiben Mitnehmereinrichtungen aufweisen, die den Abfall in einen Spalt benachbarter Walzen mitführen. Bevorzugt kann die Zerkleinerungseinrichtung wenigstens eine Shredderwalze aufweisen. Die Zerkleinerungseinrichtung kann auch durch sich horizontal bewegende und sich überschneidende Messer gebildet sein.

Um den Abfall zu desinfizieren oder um andere Flüssigkeiten zuzusetzen, die gegebenenfalls Bestandteile des Abfalls lösen, können Abgabedüsen für eine Flüssigkeit im Verdichtungsraum und/oder im Bereich der Presseinrichtung und/oder der Verdichtungseinrichtung und/oder der Zerkleinerungseinrichtung angeordnet sein. Durch diese Düsen wird die entsprechende Flüssigkeit, wie eine Desinfektionsflüssigkeit, auf den Abfall gesprüht.

Um die UV-Lampen und deren Strahlung besser ausnutzen zu können, können Wände des Eingaberaums und/oder des Verdichtungsraums und/oder der Presseinrichtung und/oder der Verdichtungseinrichtung und/oder der Zerkleinerungseinrichtung UV-reflektierend ausgebildet sein.

Beispiele in diesem Zusammenhang sind ein Polieren der Wände zur UV-Reflektion oder eine Oberflächenbehandlung der Wände, wobei die Oberflächenbehandlung auch das Aufbringen von spiegelglatten Materialien beinhaltet.

Um möglichst viel Strahlung der UV-Lampen in Richtung Zerkleinerungseinrichtung durchzulassen und damit die Wirkung der Strahlung voll ausnutzen zu können, können Presselement und/oder Verdichtungsplatte aus UV-durchlässigem Material gebildet sein. Ein Beispiel für ein solches Material ist Quarz.

Nach der Zerkleinerung des Abfalls gelangt dieser in die Sterilisiereinrichtung. Die Sterilisation kann beispielsweise durch Erhitzen des Abfalls erfolgen. Soll eine Erhitzung insbesondere der flüssigen Bestandteile des Abfalls - u.a. Wasser - auf eine höhere Temperatur als die Siedetemperatur der Flüssigkeit möglich sein, ist es notwendig, dass die Sterilisiereinrichtung einen unter Druck setzbaren Aufnahmeraum aufweist. In diesem kann dann der Abfall mit in ihm enthaltener Flüssigkeit oder auch mit der über die Abgabedüsen zugesetzten Flüssigkeit bis zu einer relativ hohen Temperatur erhitzt werden, wobei ein erhöhter Druck im Aufnahmeraum liegt. Als Beispiel sei eine Temperatur von 150 bis 180°C genannt, die in etwa einem Druck von 5 bis 7 bar entspricht.

Der Abfall wird dieser Temperatur und dem Druck eine gewisse Zeit ausgesetzt und dann wird der Druck im Raum wieder auf Umgebungsdruck vermindert und der Raum entleert. Eine einfache Möglichkeit des Aufheizens des Abfalls kann darin gesehen werden, dass Mikrowellen in den Aufnahmeraum einspeisbar sind. Dies kann durch entsprechend Magnetrone erfolgen, wie sie aus im Handel erhältlichen Mikrowellenöfen bekannt sind.

Um den möglicherweise noch erhitzten Abfall in einfacher Weise an die Verpackungseinrichtung zu überführen, kann der Aufnahmeraum eine Auswerfeinrichtung zur Übergabe des behandelten Abfalls an die Verpackungseinrichtung aufweisen. Die Auswerfeinrichtung kann als Schieber, als Transportband oder dergleichen ausgebildet sein.

Um den behandelten und sterilisierten Abfall in einfacher Weise zu sammeln und abtransportieren zu können, kann die Verpackungseinrichtung wenigstens einen Behälter zur Aufnahme des Abfalls aufweisen, der nach Befüllung verschließbar und/oder kennzeichenbar ist. Die Kennzeichnung kann u.a. Datum, Uhrzeit, Sterilisierungsparameter, Gewicht des Behälters oder dergleichen betreffen. Der verschlossene Behälter ist dann entweder automatisch aus der erfindungsgemäßen Vorrichtung abgebbar oder wird manuell aus der Vorrichtung gegebenenfalls nach Anzeige einer entsprechenden Meldung an der Vorrichtung entnommen.

Im Folgenden wird ein vorteilhaftes Ausführungsbeispiel der Erfindung anhand der einzigen Figur dargestellt. Es zeigt:

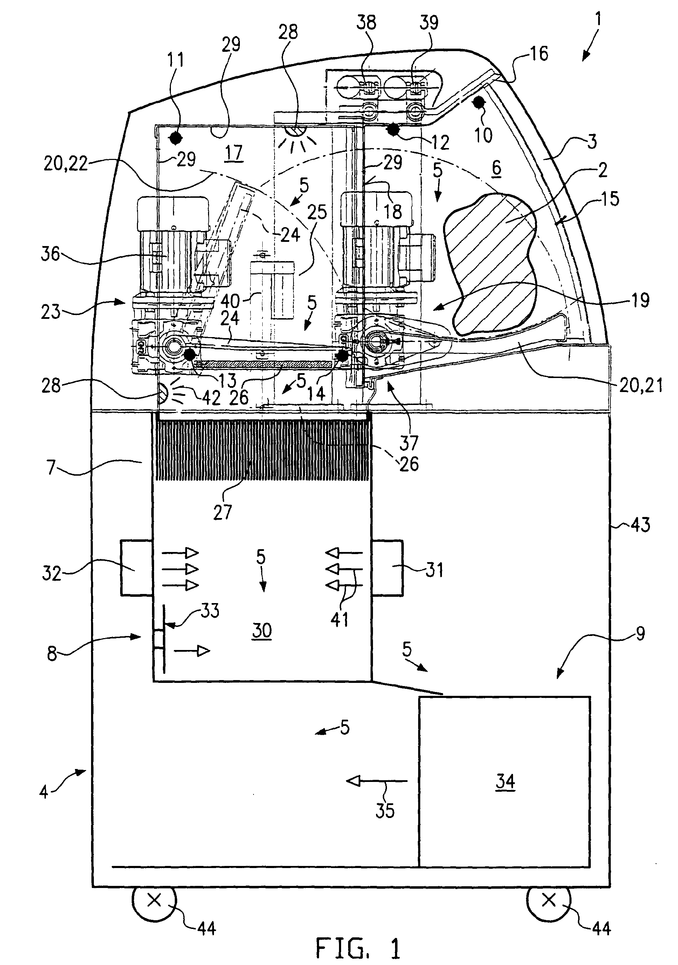

- Fig. 1: einen Vertikalschnitt durch ein Ausführungsbeispiel einer erfindungsgemäßen Vorrichtung zur Zerkleinerung und Sterilisation von insbesondere medizinischem Abfall.

In Fig. 1 ist ein Vertikalschnitt durch eine erfindungsgemäße Vorrichtung 1 zur Zerkleinerung und Sterilisierung von medizinischem Abfall 2 dargestellt. Die Vorrichtung 1 weist ein Gehäuse 43 auf, das auf seiner Unterseite fahrbar auf Rollen 44 gelagert ist.

In dem Gehäuse ist auf einer Seite eine Eingabeöffnung 3 angeordnet, die durch eine Zugangstür 15 in Form einer Schiebetür zugänglich ist. Die Zugangstür 15 öffnet sich automatisch, wenn ein im Bereich der Tür angeordneter Anwesenheitssensor 16 die Anwesenheit eines Benutzers in einem bestimmten Bereich vor der Zugangstür 15 erfasst.

Bei geöffneter Zugangstür 15 kann medizinischer Abfall 2 in einen Eingaberaum 6 der Vorrichtung 1 eingestellt werden. Anschließend schließt sich die Zugangstür 15 automatisch.

In der Folge wird der medizinische Abfall 2 entlang eines Verarbeitungspfades 5 transportiert und bearbeitet und schließlich verpackt an einer im unteren Bereich der Vorrichtung 1 angeordneten Ausgabeöffnung 4 wieder abgegeben.

Der Eingaberaum 6 ist benachbart zu einem Verdichtungsraum 17 angeordnet. Zwischen diesen ist eine Zwischentür 18 ebenfalls als Schiebetür und automatisch betätigbar angeordnet. Bei geöffneter Zwischentür 18 ist der medizinische Abfall 2 mittels einer Transfereinrichtung 19 aus dem Eingaberaum 6 in den Verdichtungsraum 17 transportierbar. Die Transfereinrichtung 19 weist insbesondere eine Kippebene 20 auf, die durch einen Kippantrieb 37 verschwenkbar ist.

In Fig. 1 ist die Kippebene 20 in einer Aufnahmestellung 21 angeordnet. Eine entsprechende Kippstellung 22 der Kippebene 20 ist gestrichelt dargestellt. Ist die Kippebene 20 in ihrer Kippstellung 22 fällt der medizinische Abfall 2 in Richtung einer Zerkleinerungseinrichtung 7. Oberhalb dieser Zerkleinerungseinrichtung ist eine Presseinrichtung 23 angeordnet. Diese weist ein verschwenkbares Element 24 und eine zugeordnete Antriebseinrichtung 36 auf. Das Presselement 24 ist in Richtung Zerkleinerungseinrichtung 7 nach unten verschwenkbar und drückt den medizinischen Abfall 2 in Richtung dieser Einrichtung. Weiterhin ist der Zerkleinerungseinrichtung 7 eine Verdichtereinrichtung 25 vorgeordnet. Diese weist eine Verdichtungsplatte 26 auf, die in vertikaler Richtung durch einen entsprechenden Hebeantrieb 40 verstellbar ist. In Fig. 1 ist die Verdichtungsplatte 26 auch in ihrer in Richtung Zerkleinerungseinrichtung 7 abgesenkten Stellung gestrichelt dargestellt.

Oberhalb von Eingaberaum 6 und Verdichtungsraum 17 sind die Türantriebe 38 und 39 angeordnet, durch die die Schiebetüren 3, 18 betätigbar sind.

Beispielhaft sind zwei Abgabedüsen 28 im Verdichtungsraum 17 dargestellt, über die beispielsweise eine Desinfektionsflüssigkeit dem medizinischen Abfall 2 zusetzbar ist.

Im Bereich von Eingaberaum 6 und Verdichtungsraum 17 sind eine Reihe von UV-Lampen 10, 11, 12, 13 und 14 angeordnet. Die UV-Lampen 10 und 12 bestrahlen den medizinischen Abfall 2 im Eingaberaum 6 nach Schließen der Zugangstür 15. Die UV-Lampe 11 bestrahlt den medizinischen Abfall nach Übergabe in den Verdichtungsraum 17 und die UV-Lampen 13 und 14 bestrahlen den medizinischen Abfall bei seiner Vorverdichtung, Komprimierung und anschließenden Zerkleinerung durch die Zerkleinerungseinrichtung 7.

Die Zerkleinerungseinrichtung 7 weist wenigstens eine Shredderwalze 27 auf, die durch eine Vielzahl von um eine Achse drehbaren Messerscheiben gebildet ist. Während der Zerkleinerung durch die Shredderwalze 27 und gegebenenfalls durch weitere zugeordnete Walzen erfolgt die Bestrahlung mit UV-Licht.

Anschließend gelangt der zerkleinerte und durchmischte sowie bereits weitgehend desinfizierte medizinische Abfall in einen Aufnahmeraum 30 einer Sterilisiereinrichtung 8. Der Aufnahmeraum 30 ist schließbar und unter Druck setzbar. Nach Verschließen des Aufnahmeraums 30 ist der eingefüllte Abfall mittels Mikrowellen-Magnetrons 31, 32 und durch entsprechende Mikrowellen 41 erhitzbar. Nach Erreichen einer bestimmten Temperatur und nach Erhitzen des medizinischen Abfalls für eine bestimmte Zeit ist dieser mittels einer im Aufnahmeraum 30 angeordneten Auswurfeinrichtung 33 in Richtung Verpackungseinrichtung 9 transportierbar. Dazu weist der Aufnahmeraum 30 an entsprechender Stelle eine Öffnung auf, die automatisch geöffnet und verschlossen werden kann.

Nach Füllen eines Behälters 34 der Verpackungseinrichtung 9 ist dieser in Abgaberichtung 35 zur Ausgabeöffnung 4 der Vorrichtung 1 transportierbar. Dort kann der Behälter entweder manuell entnommen oder automatisch ausgestoßen werden.

## Patentansprüche

1. Vorrichtung (1) zum Zerkleinern und Sterilisieren von insbesondere medizinischem Abfall (2) mit einer Eingabe- und einer Ausgabeöffnung (3, 4) und einem zwischen diesen zu durchlaufenden Verarbeitungspfad (5), entlang welchem nacheinander wenigstens ein Eingaberaum (6), eine Zerkleinerungseinrichtung (7), eine Sterilisiereinrichtung (8) und eine Verpackungseinrichtung (9) angeordnet sind, **dadurch gekennzeichnet, dass** eine Anzahl von UV-Lampen (10, 11, 12, 13, 14) entlang des Verarbeitungspfades (5) angeordnet sind, von denen wenigstens eine UV-Lampe (13, 14) die Zerkleinerungseinrichtung (7) beleuchtet.

2. Vorrichtung nach Anspruch 1, **dadurch gekennzeichnet, dass** die Eingabeöffnung (3) durch eine Zugangstür (15) verschließbar ist.

3. Vorrichtung nach Anspruch 1 oder 2, **dadurch gekennzeichnet, dass** die Zugangstür (15) automatisch öffnet und schließt.

4. Vorrichtung nach wenigstens einem der vorangehenden Ansprüche, **dadurch gekennzeichnet, dass** der Zugangstür eine Zugangsberechtigungseinrichtung zum Auslösen eines Öffnens der Tür zugeordnet ist.

5. Vorrichtung nach wenigstens einem der vorangehenden Ansprüche, **dadurch gekennzeichnet, dass** der Zugangstür (15) ein Anwesenheitssensor (16) zugeordnet ist.

6. Vorrichtung nach wenigstens einem der vorangehenden Ansprüche, **dadurch gekennzeichnet, dass** dem Eingaberaum (6) ein Verdichtungsraum (17) nachgeordnet ist.

7. Vorrichtung nach wenigstens einem der vorangehenden Ansprüche, **dadurch gekennzeichnet, dass** zwischen Eingabe- und Verdichtungsraum (6, 17) eine automatisch betätigbare Zwischentür (18) angeordnet ist.

8. Vorrichtung nach wenigstens einem der vorangehenden Ansprüche, **dadurch gekennzeichnet, dass** Zugangstür (15) und/oder Zwischentür (18) als Schiebetüren ausgebildet sind.

9. Vorrichtung nach wenigstens einem der vorangehenden Ansprüche, **dadurch gekennzeichnet, dass** Eingaberaum (6) und Verdichtungsraum (17) nebeneinander angeordnet sind.

10. Vorrichtung nach wenigstens einem der vorangehenden Ansprüche, **dadurch gekennzeichnet, dass** eine Transfereinrichtung (19) zum Transport der Abfälle vom Eingaberaum (6) zum Verdichtungsraum (17) vorgesehen ist.

11. Vorrichtung nach wenigstens einem der vorangehenden Ansprüche, **dadurch gekennzeichnet, dass** die Transfereinrichtung (19) eine zwischen einer Aufnahmestellung (21) und einer Kippstellung (22) verschwenkbare Kippebene (20) aufweist.

12. Vorrichtung nach wenigstens einem der vorangehenden Ansprüche, **dadurch gekennzeichnet, dass** eine Presseinrichtung (23) im Verdichtungsraum (17) angeordnet ist.

13. Vorrichtung nach wenigstens einem der vorangehenden Ansprüche, **dadurch gekennzeichnet, dass** die Presseinrichtung (23) wenigstens ein verschwenkbares, insbesondere plattenförmiges Presselement (14) aufweist, welches den Abfall in Richtung Zerkleinerungseinrichtung (7) drückt.

14. Vorrichtung nach wenigstens einem der vorangehenden Ansprüche, **dadurch gekennzeichnet, dass** eine Verdichtereinrichtung (25) zur Komprimierung von in Richtung Zerkleinerungseinrichtung (7) durch die Presseinrichtung (23) gedrückten Abfall der Zerkleinerungseinrichtung vorgeordnet ist.

15. Vorrichtung nach wenigstens einem der vorangehenden Ansprüche, **dadurch gekennzeichnet, dass** die Verdichtereinrichtung (25) eine in Richtung Zerkleinerungseinrichtung (7) bewegbare Verdichtungsplatte (26) aufweist.

16. Vorrichtung nach wenigstens einem der vorangehenden Ansprüche, **dadurch gekennzeichnet, dass** Verdichtereinrichtung (25) und Zerkleinerungseinrichtung (7) im Wesentlichen vertikal übereinander angeordnet sind und die Verdichtungsplatte (26) vertikal bewegbar ist.

17. Vorrichtung nach wenigstens einem der vorangehenden Ansprüche, **dadurch gekennzeichnet, dass** die Zerkleinerungseinrichtung (7) wenigstens eine Shredderwalze (27) aufweist.

18. Vorrichtung nach wenigstens einem der vorangehenden Ansprüche, **dadurch gekennzeichnet, dass** Abgabedüsen (28) für eine Flüssigkeit im Verdichtungsraum (17) und/oder im Bereich der Presseinrichtung (23) und/oder der Verdichter (25) und/oder der Zerkleinerungseinrichtung (7) angeordnet sind.

19. Vorrichtung nach wenigstens einem der vorangehenden Ansprüche, **dadurch gekennzeichnet, dass** Wände (29) des Eingaberaums und/oder des Verdichtungsraums (17) und/oder der Presseinrichtung (23) und/oder der Verdichtereinrichtung (25) und/oder der Zerkleinerungseinrichtung (7) UV-reflektierend ausgebildet sind.

20. Vorrichtung nach wenigstens einem der vorangehenden Ansprüche, **dadurch gekennzeichnet, dass** die Wände (29) zur UV-Reflektion poliert sind.

21. Vorrichtung nach wenigstens einem der vorangehenden Ansprüche, **dadurch gekennzeichnet, dass** die Wände (29) zur UV-Reflektion oberflächenbehandelt sind.

22. Vorrichtung nach wenigstens einem der vorangehenden Ansprüche, **dadurch gekennzeichnet, dass** Presselement (24) und/oder Verdichtungsplatte (26) aus UV-durchlässigem Material gebildet sind.

23. Vorrichtung nach wenigstens einem der vorangehenden Ansprüche, **dadurch gekennzeichnet, dass** die Sterilisiereinrichtung (8) einen unter Druck setzbaren Aufnahmeraum (30) aufweist.

24. Vorrichtung nach wenigstens einem der vorangehenden Ansprüche, **dadurch gekennzeichnet, dass** Mikrowellen in den Aufnahmeraum (30) einspeisbar sind.

25. Vorrichtung nach wenigstens einem der vorangehenden Ansprüche, **dadurch gekennzeichnet, dass** der Aufnahmeraum (30) eine Auswerfeinrichtung (33) zur Übergabe des behandelten Abfalls an die Verpackungseinrichtung (9) aufweist.

26. Vorrichtung nach wenigstens einem der vorangehenden Ansprüche, **dadurch gekennzeichnet, dass** die Verpackungseinrichtung (9) wenigstens einen Behälter (34) zur Aufnahme des Abfalls aufweist, der nach Befüllung verschließbar und/oder kennzeichenbar ist.
